(19) Europäisches Patentamt
European Patent Office
Office européen des brevets

(11) Publication number: **0 351 685**
**A2**

(12) **EUROPEAN PATENT APPLICATION**

(21) Application number: 89112590.8

(51) Int. Cl.4: C07K 9/00 , C07K 1/00

(22) Date of filing: 10.07.89

(30) Priority: 18.07.88 GB 8817052

(43) Date of publication of application:
24.01.90 Bulletin 90/04

(84) Designated Contracting States:
AT BE CH DE ES FR GB GR IT LI LU NL SE

(71) Applicant: GRUPPO LEPETIT S.P.A.
23, Via G. Murat
I-20159 Milano(IT)

(72) Inventor: Malabarba, Adriano
5/A Via Roma
I-20082 Binasco (MI)(IT)

(54) **Process for preparing n15-alkyl derivatives of teicoplanin compounds.**

(57) This invention relates to a process for selectively preparing $N^{15}$-alkyl derivatives of teicoplanin compounds which comprises addition of an alkali metal borohydride before adding an excess of a carbonyl compound and reacting the resulting solution with a reducing agent.

EP 0 351 685 A2

# PROCESS FOR PREPARING $N^{15}$-ALKYL DERIVATIVES OF TEICOPLANIN COMPOUNDS

The invention relates to a process for preparing $N^{15}$-alkyl derivatives of teicoplanin compounds having the following formula:

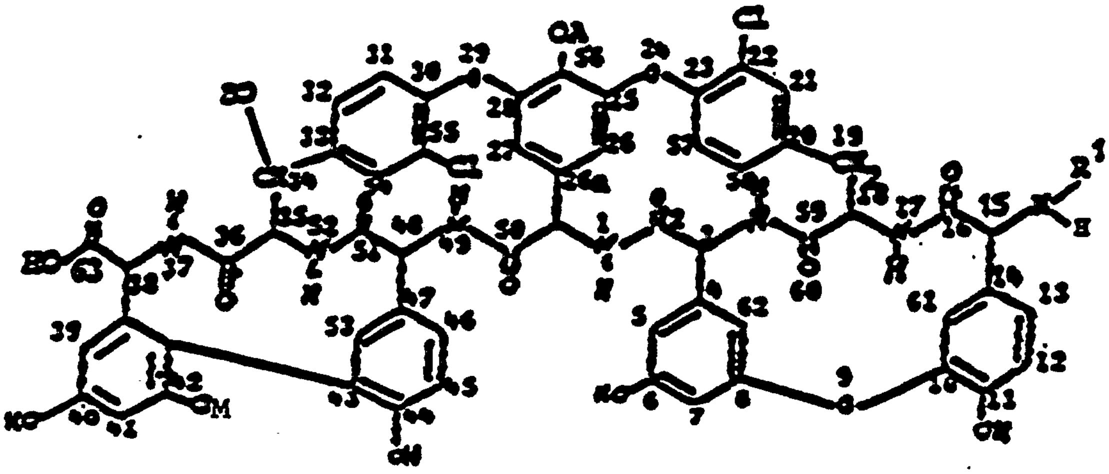

I

wherein

$R^1$ represents hydrogen, $(C_1-C_{12})$alkyl, $(C_4-C_7)$cycloalkyl, cyano $(C_1-C_3)$alkyl, $-(CH_2)_n-OOC-(C_1-C_6)$alkyl, wherein n is an integer selected from 1, 2, 3 and 4; phenyl $(C_1-C_4)$alkyl wherein the phenyl group is optionally substituted in the position ortho, meta and/or para with 1 to 3 groups selected from $(C_1-C_4)$alkyl nitro, halogen, $(C_1-C_4)$alkoxy, and phenyl;

A represents hydrogen or $-N[(C_9-C_{12})$aliphatic acyl]-beta-D-2-deoxy-2-amino-glucopyranosyl;
B represents hydrogen or N-acetyl-beta-D-2-deoxy-2-amino-glucopyranosyl;
M represents hydrogen or alpha-D-mannopyranosyl; with the proviso that B represents hydrogen only when A and M are simultaneously hydrogen;
and the addition salts thereof,
by reductive alkylation of a selected compound of formula I wherein $R^1$ is a hydrogen atom with a carbonyl compound in the presence of an alkali metal borohydride in an organic polar solvent under pre-established pH conditions which can range from neutral to basic values.

Teicoplanin is the international non-proprietary name (INN) of the antibiotic substance formerly named teichomycin which is obtained by cultivating the strain Actinoplanes teichomyceticus nov. sp. ATCC 31121 in a culture medium containing assimilable sources of carbon, nitrogen and inorganic salts (see U.S. Patent No. 4,239,751).

According to the procedure described in the above cited patent an antibiotic complex containing Teichomycin $A_1$, $A_2$ and $A_3$ is recovered from the separated fermentation broth by extraction with a suitable water insoluble organic solvent and precipitation from the extracting solvent according to common procedures.

Teichomycin $A_2$, which is the major factor of the isolated antibiotic complex, is then separated from the other factors by means of column chromatography on Sephadex$^R$.

British Patent No. 2121401 discloses that antibiotic Teichomycin $A_2$ actually is a mixture of five closely related co-produced main components.

It is possible to represent teicoplanin $A_2$ (formerly Teichomycin $A_2$) main components 1, 2, 3, 4 and 5 by the above formula I wherein $R^1$ is hydrogen, A represents $-N[(C_{10}-C_{11})$aliphatic acyl]-beta-D-2-deoxy-2-amino-glucopyranosyl, B represents N-acetyl-beta-D-2-deoxy-2-amino-glucopyranosyl, M represents alpha-D-mannopyranosyl.

More particularly, in teicoplanin $A_2$ component 1, the $[(C_{10}-C_{11})$-aliphatic acyl] substituent represents Z-4-decenoyl, in teicoplanin $A_2$ component 2 represents 8-methyl-nonanoyl, in teicoplanin $A_2$ component 3 represents decanoyl, in teicoplanin $A_2$ component 4 represents 8-methyldecanoyl, in teicoplanin $A_2$ component 5 represents 9-methyldecanoyl.

All the sugar moieties, when present, are linked to the teicoplanin nucleus through O-glycosidic bonds.

European Patent application Publication No. 306645 describes production of teicoplanin compounds where the aliphatic acyl group of the beta-D-2-deoxy-2-amino glycopyranosyl moiety is a 6-methyloctanoyl (compound A or RS3) or a n-nonanoyl radical (compound B or RS4).

In the paper entitled: "Isolation by HPLC and structural determination of minor components of teicoplanin" given by Zanol et al., at the 17th International Symposium on Chromatography, Wien, September 25-30, 1988, other two teicoplanin compounds (RS1 and RS2) are described.

Said compounds are characterized in that the aliphatic acyl moieties of the beta-D-2-deoxy-2-aminoglucopyranosyl moiety are respectively methyl-undecanoyl and dodecanoyl.

In addition, it has been found that it is possible to transform teicoplanin, a pure factor thereof or a mixture of any of said factors in any proportion, into unitary antibiotic products by means of selective hydrolysis of one or two sugar moieties.

They are named antibiotic L 17054 and antibiotic L 17046 and are described in European Patent Application Publication No. 119575 and European Patent Application Publication No. 119574, respectively.

Preferred hydrolysis conditions for the production of antibiotic L 17054 are: 0.5 N hydrochloric acid at a temperature between 70 °C and 90 °C and for a time which is generally between 15 and 90 min.

Antibiotic L 17054 is represented by the above formula I wherein $R^1$ and A represent hydrogen, B represents N-acetyl-beta-D-2-deoxy-2-amino-glucopyranosyl, M represents alpha-D-mannopyranosyl wherein the sugar moieties are linked to the peptidic nucleus through an O-glycosidic bond.

Preferred hydrolysis conditions for the preparation of antibiotic L 17046 are: 1-3 N hydrochloric acid, at a temperature between 50° and 90 °C and for a time which is generally between 30 and 60 min.

Antibiotic L 17046 is represented by the above formula I wherein $R^1$, A and M represent hydrogen atoms, and B is N-acetyl-beta-D-2-deoxy-2-aminoglucopyranosyl wherein the sugar moiety is linked to the peptidic nucleus through an O-glycosidic bond.

European Patent Application publication No. 301247 describes de-mannosyl teicoplanin derivatives, i.e. compounds of the formula I above wherein A and B are different from hydrogen.

The complete selective cleavage of all the sugar moieties of the teicoplanin compounds gives an aglycone molecule which is called antibiotic L 17392, or deglucoteicoplanin, and is represented by the above formula I wherein $R^1$, A, B, and M each individually represents a hydrogen atom. This selective hydrolysis process is described in European Patent Application Publication No 146053.

A substance having the same structural formula is disclosed in European Patent Application Publication No. 0090578 and is named antibiotic A 41030 factor B.

This substance is obtained by means of a microbiological process which involves the fermentation of the strain Streptomyces virginiae NRRL 12525 or Streptomyces virginiae NRRL 15156 in a suitable medium, the isolation, purification and separation into its components of antibiotic A 41030, an antibiotic complex of at least seven factors, antibiotic A 41030 factor B, included.

All the above named compounds, i.e. teicoplanin, teicoplanin $A_2$ complex, teicoplanin $A_2$ component 1, teicoplanin $A_2$ component 2, teicoplanin $A_2$ component 3, teicoplanin $A_2$ component 4, teicoplanin $A_2$ component 5, antibiotic L 17054, antibiotic L 17046, antibiotic L 17392, compound A or RS3 and compound B or RS4, RS1, RS2, the de-mannosyl teicoplanin derivatives of EP-A-Publication No. 301247 and any mixture thereof in any proportion, are suitable starting materials for the preparation of the alkyl derivatives of the invention.

In the present specification "teicoplanin compound" or "teicoplanin starting material" is used to indicate any one of the above starting materials, i.e. teicoplanin as obtained according to U.S. patent 4,239,751, any further purification thereof, teicoplanin $A_2$ complex, a compound of the above formula I wherein $R^1$ is hydrogen, A represents hydrogen or -N[($C_9$-$C_{12}$)aliphatic acyl]-beta-D-2-deoxy-2-amino-glucopyranosyl, B represent hydrogen or N-acetyl-beta-D-2-deoxy-2-amino-glucopyranosyl, M represents hydrogen or alpha-D-mannopyranosyl, with the proviso that B may represent hydrogen only when A and M are simultaneously hydrogen; a salt thereof, or a mixture thereof in any proportion.

As used herein the term "alkyl", either alone or in combination with other substituents, includes both straight and branched hydrocarbon groups; more particularly, "($C_1$-$C_6$)alkyl" represents a straight or branched aliphatic hydrocarbon chain of 1 to 6 carbon atoms such as methyl, ethyl, propyl, 1-methylethyl, butyl, 1-methylpropyl, 1,1-dimethylethyl, pentyl, 1-methylbutyl, 2-methylbutyl, 1-hexyl, 2-hexyl, 3-hexyl, 3,3-dimethyl-1-butyl, 4-methyl-1-pentyl and 3-methyl-1-pentyl; likewise, "($C_1$-$C_4$)alkyl" represents a straight or branched hydrocarbon chain of 1 to 4 carbon atoms like those exemplified above.

The term "halogen" represents an halogen atom selected from chlorine, bromine and iodine.

The compounds of the invention contain acid and basic functions and can form, in addition to "internal salts", acid and base addition salts according to conventional procedures.

For the scope of the present description and claims the "internal salts" are encompassed by the

definition of "non-salt" or "non-addition salt" form.

Preferred addition salts are the pharmaceutically acceptable acid and/or base addition salts.

With the term "pharmaceutically acceptable acid and/or base addition salts" are intended those salts with acids and/or bases which from biological, manufacturing and formulation standpoint are compatible with the pharmaceutical practice as well as with the use in the animal growth promotion.

Representative and suitable acid addition salts of the compounds of formula I include those salts formed by standard reaction with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, glutamic, camphoric, glutaric, glycolic, aspartic, phthalic, tartaric, lauric, stearic, salicylic, methanesulfonic, benzenesulfonic, sorbic, picric, benzoic, cinnamic and the like acids and salts with aminoacids like lysine, arginine, asparagine, glutamine, hystidine, serine, methionine, leucine and the like.

Representative examples of these bases are: alkali metals or alkaline-earth metal hydroxides such as sodium, potassium, and calcium hydroxide; and organic aliphatic, alicyclic or aromatic amines such as methylamine, dimethylamine, trimethylamine, and picoline.

A preferred group of compounds prepared with the process of the present invention is represented by those compounds of formula I wherein $R^1$ represents $(C_1\text{-}C_{12})$alkyl, $(C_4\text{-}C_6)$cycloalkyl, cyano-$(C_1\text{-}C_2)$alkyl, $(CH_2)_nOOC(C_1\text{-}C_3)$alkyl wherein n represents 1 or 2, phenyl$(C_1\text{-}C_3)$alkyl wherein the phenyl moiety is optionally substituted with 1 or 2 substituents selected from methyl, nitro, bromo, chloro, iodo or methoxy.

Another preferred group of compounds prepared by means of the process of the present invention is represented by those compounds of formula I wherein $R^1$ represents $(C_1\text{-}C_4)$alkyl and A, B, and M are as defined.

A further preferred group of compounds which can be prepared with the process of the invention is represented by those compounds of formula I wherein $R^1$ represents $(C_1\text{-}C_4)$alkyl and A, B, and M represent the sugar moieties, defined above or A, B, and M, represent hydrogen atoms.

The process of the present invention permits also to prepare another group of preferred compounds which is represented by those compounds of formula I wherein $R^1$ represents methyl or ethyl and A, B, and M represent the sugar moieties defined above or A, B, and M represents hydrogen atoms. Preferred compounds prepared by the process of the invention are those compounds of formula I wherein A, B and M represent the sugar moieties defined above or A, B and M represent hydrogen atoms. Further preferred compounds prepared by this process are those compounds of formula I wherein A, B and M represent the sugar moieties defined above and the $N[(C_9\text{-}C_{12})$aliphatic acyl]-substituent represents 8-methylnonanoyl.

Other preferred compounds which can be prepared with the process of the present invention are:

- a compound of formula I wherein $R^1$ represents $(C_1\text{-}C_4)$alkyl, or $(C_8\text{-}C_{12})$alkyl
- a compound of formula I wherein $R^1$ is methyl
- a compound of formula I wherein A represents hydrogen, B and M are different from hydrogen, $R^1$ represents a linear $C_{11}$ alkyl
- a compound of formula I wherein A and M represent hydrogen, B is different from hydrogen, $R^1$ represents n-octyl.

The transformation of the free amino or non-salt compounds of the invention into the corresponding addition salts, and the reverse, i.e. the transformation of an addition salt of a compound of the invention into the non-salt or free amino form, are within the ordinary technical skill and are encompassed by the present invention.

For instance, a compound of formula I can be transformed into the corresponding acid or base addition-salt by dissolving the non-salt form in an aqueous solvent and adding a slight molar excess of the selected acid or base.

The resulting solution or suspension is then lyophilized to recover the desired salt. Instead of lyophilizing, in some instances, it is possible to recover the final salt by extraction with an organic solvent, concentration to a small volume of the separated organic phase and precipitation by adding a non-solvent.

In case the final salt is insoluble in an organic solvent where the non-salt form is soluble it is recovered by filtration from the organic solution of the non-salt form after addition of the stoichiometric amount or a slight molar excess of the selected acid or base.

The non-salt form can be prepared from a corresponding acid or base salt dissolved in an aqueous solvent which is then neutralized to free the non-salt form.

This is then recovered for instance by extraction with an organic solvent or is transformed into another base or acid addition salt by adding the selected acid or base and working up as above.

When following the neutralization desalting is necessary, a common desalting procedure may be employed.

For example, column chromatography on controlled pore polydextrane resins (such as Sephadex L H 20) or silanized silica gel may be conveniently used.

After eluting the undesired salts with an aqueous solution, the desired product is eluted by means of linear gradient or step-gradient of a mixture of water and a polar or apolar organic solvent, such as acetonitrile/water from 50:50 to about 100% acetonitrile.

As is known in the art, the salt formation either with pharmaceutically acceptable acids (bases) or non-pharmaceutically acceptable acids (bases) may be used as a convenient purification technique.

After formation and isolation, the salt form of a compound of formula I can be transformed into the corresponding non-salt or into a pharmaceutically acceptable salt.

In some instances the acid addition salt of a compound of formula I is more soluble in water and hydrophilic solvents and has an increased chemical stability.

However, in view of the similarity of the properties of the compounds of formula I and their salts, what is said in the present application when dealing with the biological activities of the compounds of formula I applies also to their pharmaceutically acceptable salts, and viceversa.

The compounds prepared by means of the process of the present invention are useful as semi-synthetic antibacterial agents mainly active against gram positive bacteria or as growth promoters.

The mono-$N^{15}$-alkyl derivatives of the invention are prepared by a process comprising a reductive alkylation of a teicoplanin compound having formula I wherein $R^1$ is a hydrogen atom with a proper reactant such as a carbonyl compound in the presence of a reducing agent in a selected polar protic solvent under well established pH conditions which can range from neutral to basic values.

As known, in fact, when mixed hydrides or suitable reducing agents reduce a Schiff base in the presence of an excess of a carbonyl compound, usually the final mixture contains both di-alkyl derivatives and mono-alkyl ones. In said mixture usually the mono-alkyl derivatives are obtained in very poor yields while the di-alkyl derivatives are obtained in good yields, and it is troublesome to separate the single components.

The process for preparing selectively the $N^{15}$-mono-alkyl derivatives of teicoplanin compounds object of the present invention, comprises:

a) adding to a suspension of teicoplanin compound in an organic polar protic solvent a suitable amount of an alkali metal borohydride at a temperature comprised between 0°C and 30°C, then

b) adding a molar excess of a carbonyl compound of at least 20 times with respect to teicoplanin starting material, and

c) reacting the resulting solution with a metal alkali borohydride.

Among the organic polar protic solvents lower alkanols having from 1 to 4 carbon atoms are preferred. In particular, methanol is the preferred solvent used.

Sometimes, in particular cases, a small amount of a polar protic co-solvent can be added to completely dissolve teicoplanin starting material during the course of the reaction, e.g. N-N-dimethylformamide, 1,3-dimethyl-3,4,5,6-tetrahydro 2(1H)-pyramidone (DMPU), dimethylsulfoxide.

The temperature at which the reaction is carried out is comprised between 0°C and 30°C, preferably it ranges from 10°C to 15°C.

It is important to use a large molar excess of carbonyl compound, generally from 20 to 100 times with respect to teicoplanin starting material. The carbonyl compound used has the same carbon skeleton of substituent $R^1$ and whose oxo group is on the carbon atom which links to the $N^{15}$-amino group of teicoplanin moiety.

For example, if

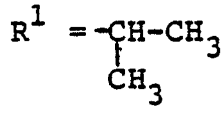

is the desired $N^{15}$monoalkyl group,
then

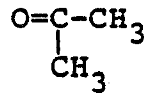

is the useful carbonyl compound which has to be employed as starting reactant.

If $R^1$ = $-CH_2CH_2CH_3$ is the desired $N^{15}$monoalkyl group, then

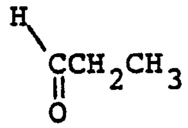

is the suitable carbonyl compound which is used as starting material.

Sodium borohydride is the preferred alkali metal borohydride used in the step a).

As metal alkali borohydrides used in the step c) among which the sodium borohydride is the preferred one, the potassium and the sodium cyano-borohydrides can be used.

According to the present invention it is important to maintain the reactants addition order as indicated above in steps a), b) and c).

In a preferred embodiment of the invention before adding the excess of carbonyl compound to the reaction mixture, the alkali metal borohydride is allowed to completely decompose under stirring. Generally it takes a time comprised between 30 minutes and 120 minutes.

In particular, the carbonyl compound can be preferably added when the reaction mixture is a clear solution.

The suitable amount of alkali metal borohydride added can be different depending on the particular teicoplanin compound used as starting material, on the solvent used and on the temperature of the reaction, but it is advisable to add an amount of alkali metal borohydride such that the pH of the reaction mixture is alkaline, preferably between pH 8 and 10, as determined after diluting a sample of the non-aqueous reaction mixture with 4 volumes of water.

The process of the invention is an important route for preparing the mono-alkyl derivatives of teicoplanin, in fact the high selectivity together with the fast conversion rate which are associated with very high yields and absence of side-reactions and by-products, allow a simple and fast "one pot" reaction leading to the desired compounds after an ordinary desalting operation thus avoiding sophisticated and expensive purification steps.

The reaction times of the process for preparing $N^{15}$-mono-alkyl derivatives of teicoplanin depend on the factors reported above such as the starting compound used, the solvent, the amount of alkali metal borohydride used and the like, and on the structure of reactant carbonyl compounds but, in general, it is preferred to leave the reaction mixture under stirring from 1 to 10 h after the addition of the reactants and before treating with the reducing agent.

In addition, the sugar moiety of an $N^{15}$-alkyl compound of formula I may be selectively removed to transform it into a different compound having the same general formula.

For example, an $N^{15}$-alkyl compound of formula I wherein A, B, and M represent a sugar moiety as above defined can be transformed into the corresponding compound wherein B and M are as above and A is hydrogen by means of controlled acid hydrolysis in a strong concentrated aqueous organic acid.

The concentrated organic acid in this case is preferably aqueous trifluoroacetic acid at a concentration between 75% and 95%, and the reaction temperature is preferably between 10° and 50° C.

The preferred hydrolysis conditions are represented by about 90% trifluoroacetic acid at room temperature.

The reaction time varies depending on the other specific reaction parameters but, in any case, the reaction may be monitored by TLC or preferably HPLC techniques.

An analogous selective hydrolysis is reported in European Patent Application Publication No. 146822.

Similarly, $N^{15}$-alkyl compounds of formula I wherein A, B, and M represent a sugar moiety as above defined or A represents hydrogen and B and M represent sugar moieties as above defined can be transformed into the corresponding alkyl compounds of formula I wherein A and M represent hydrogen and B represent a sugar moiety as defined by means of a selective hydrolysis with a strong acid in the presence of a polar aprotic solvent selected from ethers, ketones, and mixture thereof which are liquid at room temperature.

Preferred hydrolysis conditions are in this case represented by the use of a concentrated mineral acid in the presence of an ether such as dimethoxyethane at room temperature.

Also in this case, the reaction course may be monitored by TLC or preferably HPLC. An analogous selective hydrolysis is reported in European Patent Application Publication No. 175100.

According to another embodiment of the present invention, an $N^{15}$-alkyl compound of formula I wherein A, B and M represent sugar moieties as defined above, an $N^{15}$-alkyl compound of formula I wherein A

represents hydrogen and B and M represent the above defined sugar moieties, or an $N^{15}$-alkyl compound of formula I wherein A and M represent hydrogen, and B represents a sugar moiety as above defined, may be transformed into the corresponding alkyl compounds of formula I wherein A, B and M represent hydrogen atoms by means of a selective hydrolysis in an organic protic solvent selected from aliphatic acids and alpha-halogenated aliphatic acids which at the reaction temperature are liquids, aliphatic and cycloaliphatic alkanols which at the reaction temperature are liquids slightly mixable with water, phenyl-substituted lower alkanols wherein the phenyl moiety may optionally carry ($C_1$-$C_4$)alkyl, ($C_1$-$C_4$)alkoxy or halo rests which at the reaction temperature are liquids slightly mixable with water, and beta-poly-halogenated lower alkanols, which at the reaction temperature are liquids; in the presence of a strong acid, compatible with the solvent, selected from strong mineral acids, strong organic acids and strong acid cation exchanger resins in the hydrogen form and at a temperature between 20°C and 100°C.

In this case, the preferred hydrolysis conditions are represented by the use of a mineral acid, such as hydrochloric acid, in an haloalkanol such as trifluoroethanol, at a temperature between 65°C and 85°C.

Analogous selective hydrolysis conditions on a similar substrate are described in European Patent Application Publication No. 146053.

In the following table (Table I) the structure formulas of representative examples of compounds of the invention are reported.

TABLE I

| COMPOUND | A | B | M | $R^1$ | YIELD % |
|---|---|---|---|---|---|
| 1 | $-GNHCOR_{(1-5)}$ | $-GNHCOCH_3$ | -M | $CH_3$ | 95% |
| 2 | do | do | do | $C_2H_5$ | 93% |
| 3 | do | do | do | $i-C_3H_7$ | 80% |

- $GNHCOR_{(1-5)}$ represents N-/7(C$_{10}$-C$_{11}$)aliphatic acyl7-beta-D-2-deoxy-2-aminoglucopyranosyl
- $GNHCOCH_3$ represents N-acetyl-beta-D-2-deoxy-2-aminoglucopyranosyl
- $GNHCOR_{(2)}$ represents N-/8-methyl-nonanoyl7-beta-D-2-deoxy-2-aminoglucopyranosyl

continued... TABLE I

| COMPOUND | A | B | M | $R^1$ | YIELD % |
|---|---|---|---|---|---|
| 4 | $-GNHCOR_{(2)}$ | $-GNHCOCH_3$ | -M | $CH_3$ | 96% |
| 5 | H | do | do | $CH_3$ | (*) |
| 6 | do | do | H | $CH_3$ | (**) |
| 7 | do | do | do | $n-C_8H_{17}$ | 87% |

* Starting from antibiotic L 17054: 89%
Starting from compound 4: 93%

** Starting from antibiotic L 17046: 86%
Starting from compound 1: 81%

continued... TABLE I

| COMPOUND | A | B | M | $R^1$ | YIELD % |
|---|---|---|---|---|---|
| 8 | H | H | H | $CH_3$ | 84% |
| 9 | do | do | do | $C_2H_5$ | (*) |
| 10 | do | do | do | n-$C_3H_7$ | 97% |
| 11 | do | do | do | cyclo-$C_5H_9$ | 84% |

* Starting from antibiotic L 17392: 96%
Starting from compound 2: 64%

continued... TABLE I

| COMPOUND | A | B | M | $R^1$ | YIELD % |
|---|---|---|---|---|---|
| 12 | H | H | H | $-CH_2C_6H_5$ | 78% |
| 13 | $-GNHCOR_{(2)}$ | $-GNHCOCH_3$ | M | $C_2H_5$ | - |
| 14 | do | do | do | $i-C_3H_7$ | - |

HPLC Analysis

The following table (Table II) reports the $t_R$ of representative examples of the compounds of the invention.

The assays were run with a HPCL Hewlett - Packard 1084 apparatus equipped with UV detector (254 nm).

Columns:

Stainless steel Precolumn Hibar LichroCART packed with Lichrosorb RP-8 Merck (silanized silica gel 5 micrometer) followed by a Hibar LichroCART column (25 cm) pre-packet with Lichrosorb RP-8 (7 micrometer)
Flow rate : 1.5 ml/min
Injection volume : 30 microliter

Eluents:

Solution A: $CH_3CN/NaH_2PO_4$ (0.02 M), 5/95 (v/v)
Solution B: $CH_3CN/NaH_2PO_4$ (0.02 M), 75/25 (v/v)

| Gradient profile: | | | | | | |
|---|---|---|---|---|---|---|
| minute:<br>% B in A | 0<br>8 | 40<br>40 | 45<br>60 | 48<br>65 | 55<br>8 | 56<br>- |

TABLE II

| (HPLC Analysis) | | |
|---|---|---|
| No. COMPOUND | $t_R$ | $t_R/t_R$ L 17392 |
| 1 | 27.07-28.12*-28.85 | 1.84 |
| | 31.55-32.14* | 2.08 |
| 5 | 11.25 | 0.74 |
| 7 | 43.63 | 2.84 |
| 10 | 22.56 | 1.49 |
| 11 | 26.68 | 1.75 |
| Deglucoteicoplanin (standard) | 15.30 | 1.00 |

* Peak utilized for the ratio calculation.

The following table (TABLE III) reports the acid-base titration data of some representative compounds of the invention. The assays were carried out in Methylcellosolve $^R/H_2O$, 4/1 (v/v). A sample (10 micromole) is dissolved in about 20 ml, then 0.01 N HCl (2 ml) is added and the mixture is titrated with 0.01 N NaOH in the same solvent mixture.

TABLE III

| Acid base titration | | | |
|---|---|---|---|
| COMPOUND | Salt Form | $pK_1$ | $pK_2$ |
| 1 | Internal salt | 4.9 | 7.1 |
| 5 | Internal salt | 4.9 | 7.0 |

The I.R. most significant bands ($cm^{-1}$) of some compounds of the invention recorded with a Perkin-Elmer 580 spectrometer in nujol mull are reported below: (o.b. = overlapped band)

TABLE IV

IR Data ($cm^{-1}$, nujol)

| COMPOUND | $\nu$NH<br>Glycosidic and phenolic OH | $\nu$ C=0 (COOH)<br>(amide I) | $\delta$NH<br>(amide II) | Glucoside<br>$\delta$OH, $\nu$C-O | Phenolic<br>$\nu$C-O |
|---|---|---|---|---|---|
| 1 | 3700-3100 | 1725 (COOH)<br>1650 (amide I) | 1505 | 1250-1190<br>1100-930 | o.b. |
| 2 | 3700-3100 | 1640 (amide I) | 1510 | 1250-1190<br>1100-930 | o.b. |

Continued... TABLE IV

| COMPOUND | νNH Glycosidic and phenolic OH | νC=0 (COOH) (amide I) | δNH (amide II) | Glucoside δOH, νC-O | Phenolic νC-O |
|---|---|---|---|---|---|
| 3 | 3700-3100 | 1650 (amide I) | 1510 | 1250-1190<br>1100-930 | o.b. |
| 5 | 3700-3100 | 1650 (amide I) | 1515 | 1250-1190<br>1100-930 | o.b. |
| 6 | 3700-3100 | 1725 (COOH)<br>1645 (amide I) | 1515 | 1250-1190<br>1100-930 | o.b. |

Continued... TABLE IV

| COMPOUND | ν NH Glycosidic and phenolic OH | νC=0 (COOH) (amide I) | δ NH (amide II) | Glucoside δOH, νC-O | Phenolic νC-O |
|---|---|---|---|---|---|
| 7 | 3700-3100 | 1725 (COOH) 1645 (amide I) | 1515 | 1250-1190 1100-930 | o.b. |
| 8 | 3700-3100 | 1725 (COOH) 1640 (amide I) | 1510 | | 1060, 1005 |
| 9 | 3700-3100 | 1725 (COOH) 1645 (amide I) | 1510 | | 1060, 1005 |

Continued... TABLE IV

| COMPOUND | $\nu NH$ Glycosidic and phenolic OH | $\nu C=0$ (COOH) (amide I) | $\delta NH$ (amide II) | Glucoside $\delta OH$, $\nu C-O$ | Phenolic $\nu C-O$ |
|---|---|---|---|---|---|
| 10 | 3700-3100 | 1650 | 1515 | | 1005 |
| 11 | 3700-3100 | 1650 | 1515 | | 1230<br>1060, 1010 |

## TABLE V

UV-Vis data of some representative compounds of the invention recorded with a Unicam SP 500 spectrophotometer are reported below (Lambda max, nm):

| Solvent | Compound No. 1 | Compound No. 2 | Compound No. 3 |
|---|---|---|---|
| Methanol | | | |
| 0.1N HCl | 279-280 | 279 | 279 |
| Phosphate buffer pH 7.4 | 279-280 | 279 | 279 |
| Phosphate buffer pH 9.0 | 280 | | |
| 0.1N KOH | 298 | 298 | 298 |

Continued... TABLE V

| Solvent | Compound No. 5 | Compound No. 6 | Compound No. 8 | Compound No. 9 |
|---|---|---|---|---|
| Methanol | 280 | | | |
| 0.1N HCl | 278 | 279 | 279 | 279 |
| Phosphate buffer pH 7.4 | 278 | 279 | 279 | 279 |
| Phosphate buffer pH 9.0 | 280 | | | |
| 0.1N KOH | 296 | 297 | 297 | 297 |

Table VI reports $^{1}H$-NMR data obtained at 250 MHz with a Bruker AM-250 Spectrometer in DMSO-$D_6$ at 20°C, at a sample concentration of 20 mg/ml (internal standard : TMS, delta = 0.00 ppm).

TABLE VI

| COMPOUND | |
|---|---|
| 1 | 0.83, 1.13-1.22, 1.43, 2.02 (acyl chain); 1.87 (acetylglucosamine); 2.49 ($N^+CH_3$); 3.48 (mannose); 5.60 ($C-_{27}$ H); 5.10 ($C-_{26}$ H); 4.09-5.70, (peptidic CH's); 6.20-8.60 (aromatic protons and peptidic NH's) |
| 2 | 0.82, 140, 2.01 (acyl chain) 1.85 (acetylglucosamine); 1.05, 3.08 ($N-C_2H_5$); 4.11-5.61 (peptidic CH,s); 6.23-8.34 (aromatic protons and peptidic NH's |
| 3 | 0.85, 1.35, 2.03 (acyl chain); 1.86 (acetyl glucosamine); 0.97, 1.05 ($CH_3$-isopropyl) 3.48 (mannose); 5.08 ($C_{26}$-H); 5.56 ($C_{27}$-H); 6.28-8.63 (aromatic protons); 4.09-5.66 (peptidic CH's) |
| 5 | 1.86 (acetylglucosamine); 2.30 ($N-CH_3$); 4.09-5.70 (peptidic CH's 5.59 ($C-_{27}$ H); 5.10 ($C-_{26}$ H); 4.09-5.70 (peptidic CH's); 6.02-8.49, (aromatic CH's and peptidic NH's) |

Continued... TABLE VI

| COMPOUND | |
|---|---|
| 6 | 1.86 (acetylglucosamin); 2.46 ($N-CH_3$); 4.09-5.61 (peptidic CH'S); 621-8.50, (aromatic protons a peptidic NH's) |
| 7 | 0.86, 1.22, 1.62 (n-octyl chain); 1.87-3.02, 3.70 (acetylglucosamine); 4.00-5.70 (peptidic CH's); 5.51 ($C-_{27}$ H'); 6.04-8.70 (aromatic protons and peptidic NH's); 8.00-10.20 (phenolic OH's) |
| 8 | 2.48, ($N-CH_3$); 4.09-5.60 (peptidic CH's); 5.09 ($C_{26}-H$); 5.50, ($C_{27}-H$); 6.24-8.42 (aromatic protons and peptidic NH's) |
| 9 | 1.12, 2.27, ($N-C_2-H_5$), 4.09-5.62 (peptidic CH's); 5.09 ($C_{26}-H$); 5.61, ($C_{27}-H$); 6.23-8.44 (aromatic protons a peptidic NH's) |

The antibacterial activity of the compounds prepared by means of the process of the present invention can be demonstrated in vitro by means of standard agar-dilution tests.

Isosensitest broth (Oxoid) and Todd-Hewitt broth (Difco) are used for growing staphylococci and streptococci, respectively. Broth cultures are diluted so that the final inoculum is about $10^4$ colony forming units/ml (CFU/ml). Minimal inhibitory concentration (MIC) is considered as the lowest concentration which shows no visible growth after 18-24 h incubation at 37° C. The results of the in vitro antibacterial testing of representative compounds of formula I are summarized in Table VII.

TABLE VII

In vitro (MIC microgram/ml)

| Test Organism | Compound No. 1 | Compound No. 2 | Compound No. 5 |
|---|---|---|---|
| S. haemolyticus L 602 | n.d. | 16 | n.d. |
| S. aureus Tour | 0.12 | 0.5 | 0.5 |
| S. aureus Tour + 30% bovine serum | 0.25 | 1 | 2 |
| S. epidermidis ATCC 12228 | 0.25 | 0.5 | 0.5 |
| S. pyogenes C203 | 0.06 | 0.06 | 1 |
| S. pneumoniae UC41 | 0.06 | 0.12 | 1 |
| S. faecalis ATCC 7080 | 0.12 | 0.12 | 4 |
| E. coli SKF 12140 | >128 | >128 | >128 |
| P. vulgaris X19H ATCC 881 | >128 | >128 | >128 |
| P. aeruginosa ISM | >128 | >128 | >128 |

Continued... TABLE VII

| Test Organism | Compound No. 6 | Compound No. 7 | Compound No. 8 |
|---|---|---|---|
| S. haemolyticus L 602 | 0.5 | n.d. | 0.12 |
| S. aureus Tour | 0.12 | 0.06 | 0.12 |
| S. aureus Tour + 30% bovine serum | 0.5 | 0.25 | 0.12 |
| S. epidermidis ATCC 12228 | 0.06 | 0.06 | 0.016 |
| S. pyogenes C203 | 0.5 | 0.06 | 0.12 |
| S. pneumoniae UC41 | 2 | 0.06 | 0.12 |
| S. faecalis ATCC 7080 | 1 | 0.06 | 0.12 |
| E. coli SKF 12140 | >128 | >128 | >64 |
| P. vulgaris X19H ATCC 881 | >128 | >64 | >128 |
| P. aeruginosa ISM | >128 | >128 | >128 |

Continued... TABLE VII

| Test Organism | Compound No. 9 | Compound No. 10 | Compound No. 11 |
|---|---|---|---|
| S. haemolyticus L 602 | 0.25 | n.d. | n.d. |
| S. aureus Tour | 0.06 | 0.06 | 0.06 |
| S. aureus Tour + 30% bovine serum | 0.25 | 0.25 | 0.5 |
| S. epidermidis ATCC 12228 | 0.03 | 0.016 | 0.03 |
| S. pyogenes C203 | 0.06 | 0.12 | 0.12 |
| S. pneumoniae UC41 | 0.12 | 0.12 | 0.06 |
| S. faecalis ATCC 7080 | 0.12 | 0.25 | 0.25 |
| E. coli SKF 12140 | >128 | >64 | >128 |
| P. vulgaris X19H ATCC 881 | >128 | >128 | >128 |
| P. aeruginosa ISM | >128 | >128 | >128 |

The $ED_{50}$ values (mg/Kg) of representative compounds prepared by the process of the invention in vivo tests in mice experimentally infected with S. pyogenes L 49 according to the procedure described by V. Arioli et al., Journal of Antibiotics 29, 511 (1976) are in the range of 0.17-5.0 mg/kg s.c., and mainly between 0.17 and 1.25 mg/Kg s.c., and in the range 170-300 mg/Kg or more per os.

In view of the above reported antimicrobial activity, the compounds prepared according to the present invention can effectively be employed as the active ingredients of antimicrobial preparations used in human and veterinary medicine for the prevention and treatment of infectious diseases caused by pathogenic bacteria which are susceptible to said active ingredients.

In such treatments, these compounds may be employed as such or in the form of mixtures in any proportion or pharmaceutical composition in admixture with the known pharmaceutically acceptable carriers or excipients.

The compounds prepared by the process of the present invention can be administered orally, topically or parenterally wherein however, the parenteral administration is preferred.

Depending on the route of administration, these compounds can be formulated into various dosage forms.

Preparations for oral administration may be in the form of capsules, tablets, liquid solutions or suspensions which can be prepared according to known per se techniques.

The compound prepared according to the process of the invention are generally effective at a dosage comprised between about 0.5 and about 30 mg of active ingredient per Kg of body weight, preferably divided in 2 to 4 administrations per day.

Besides their activity as medicaments, the compounds prepared according to the present invention can be used as animal growth promoters preferably in admixture with conventional carrier or feed.

The following examples further illustrate the invention and must not be construed as limiting it.

## Example 1

### Preparation of Compound 1

To a stirred suspension of 60 g (about 30 mmol) of teicoplanin $A_2$ complex in 1.5 L of methanol, 60 g (about 1.62 mol) of sodium borohydride is added portionwise while maintaining the temperature at about 45 °C.

After about 90 min, 15 ml (about 200 mmol) of 40% (by weight) aqueous formaldehyde is added at room temperature and stirring is continued for 60 min. The reaction mixture is then cooled at 5 °C. and 30 g (about 0.81 mol) of sodium borohydride is added in one portion, while cooling at 10-15 °C.

After about 30 min, 152 ml (about 2.64 mol) of glacial acetic acid is dropped at room temperature and the resulting solution is evaporated at 40 °C under reduced pressure. The residue is re-dissolved in 1.5 L of water and loaded at the top of a column of 2.4 g of silanized Silica-gel (particle size 0.06-0.2 mm; Merck Co.) in water. The column is developed with 3 L of water, then with 3 L of a mixture acetonitrile:water 2:8 (v/v), with 3 L of acetonitrile:water 1:1 (v/v), and finally with 7 L of a 1:1 (v/v) mixture acetonitrile:water adjusted to pH 3.0 with glacial acetic acid, while collecting 200 ml fractions, which are monitored by HPLC.

Those fractions containing pure title compound are pooled and enough n-butanol is added to obtain, after concentration of the resulting mixture at 40 °C under vacuum, a dry butanolic suspension of about 300 ml.

On adding 700 ml of ethyl ether, a solid separates which is collected by filtration, washed with 300 ml of ethyl ether and dried in vacuo at room temperature overnight to yield 58.7 g (95%) of pure Compound 1, as the acetate.

## Example 2

### Preparation of Compound 2

By following the same procedure as described in Example 1 but using 10 ml (about 288 mmol) of acetaldehyde as the reactant carbonyl compound, from 60 g of teicoplanin $A_2$ complex, 57.5 g (93%) of

Compound 2 is obtained, as the acetate.

Example 3

Preparation of Compound 3

By substantially following the same procedure as described in the Example 1 above, but using acetone (138 ml; about 3 mol) instead of formaldehyde, 49.4 g (80%) of Compound 3 is obtained, as the acetate from 60 g of teicoplanin $A_2$ complex. In this case, after the addition of acetone, the reaction mixture is stirred at room temperature for 20 h before treating with sodium borohydride.

Example 4

Preparation of Compound 4

By following substantially the same procedure as described above in Example 1, but starting from 9.5 g (about 5 mmol) of the component "2" of teicoplanin $A_2$, 9.15 g (96%) of the compound 4 is obtained. In this case, the compound of the title is obtained as the internal salt by working up the eluates of the column chromatography step according to a procedure which includes pooling the fractions which contains pure Compound 4 and adjusted at pH 5.8 with 1N sodium hydroxide. Then, the resulting suspension is concentrated to a final volume of about 150 ml and by filtration a solid precipitated is collected. Then it is washed with 50 ml of water and dried in vacuo at 40 °C (over phosphorus pentoxyde).

Example 5

Preparation of Compound 5

By following the above procedure of the Example 1, but starting from 16 g (about 10 mmol) of antibiotic L 17054, 14.2 g (89%) of Compound 5 is obtained, as the internal salt.

It is possible to obtain Compound 5 also starting from the Compound 4 according to the following procedure.

A solution of 1 mmol of Compound 4 in 50 ml of 90% aqueous trifluoroacetic acid (TFA) is stirred at room temperature for 90 min, then the solvent is completely evaporated at 30 °C under vacuum.

The obtained oily residue is triturated with ethyl ether, the solid which form is collected, washed with ethyl ether and dried in vacuo overnight at 30 °C, yielding crude Compound 5, as the trifluoroacetate.

This crude compound is dissolved in 100 ml of a mixture $CH_3CN$:$H_20$, 1:1 (v/v) and the resulting solution is adjusted to pH 6.3 with 1N NaOH.

Most of the acetonitrile is then evaporated at room temperature under vacuum to obtain an aqueous suspension which is adjusted to pH 5.6 with 0.1N HCl.

The solid which precipitates is collected by filtration, washed with $H_2O$ and dried at 40 °C in vacuo (over $P_2O_5$) overnight, giving Compound 5 as the internal salt. This internal salt may be transformed into the corresponding hydrochloride by dissolving it (0.5 mmol) in a mixture of (60 ml) $CH_3CN$ : 005 N HCl : n-$C_4H_9OH$, 1:1:2 (v/v/v), under stirring at room temperature.

After concentration to a small volume (about 5 ml) at 40 °C under vacuum, ethyl ether (about 25 ml) is added and the solid which separates is collected by filtration, washed with ethyl ether and dried in vacuo overnight at 40 °C, thus yielding (about 93%) of Compound 5 as the hydrochloride.

Example 6

Preparation of Compound 6

By following the procedure of Example 1, but starting from 14.5 g (about 10 mmol) of antibiotic L 17046, 12.5 g (86%) of compound 6 is obtained, as the internal salt.

It is possible to obtain Compound 6 also starting from the Compound 1 according to the following procedure.

A stirred suspension of 3 g (about 1.5 mmol) of Compound 1 in 100 ml of a saturated solution of HCl in 1,2-dimethoxyethane is heated at 50°C for 3 h, while bubbling dry HCl. The solvent is evaporated at 50°C under reduced pressure and the residue is dissolved in 200 ml of $H_2O/CH_3CN$, 95/5. The resulting solution is adjusted to pH 3.6 with 1 N NaOH and loaded on a column of 150 g of silanized Silica-gel (0.06-0.2 mm, Merck Co.) in $H_2O$. The column is developed with a linear gradient from 5% to 30% of $CH_3CN$ in $H_2O$, in 15 h at the rate of 200 ml/h, while collecting 15 ml fractions, which are checked by HPLC. Those fractions containing the pure title compound are combined and concentrated at 40°C under vacuum in the presence of n-butanol obtaining a dry butanolic suspension (about 30 ml) to which ethyl ether (about 70 ml) is added. The solid which separates is collected, washed with ethyl ether and dried at 40°C in vacuo overnight, yielding (81%) of Compound 6, as the hydrochloride.

<u>Example 7</u>

Preparation of Compound 7

By essentially following the procedure of Example 3, but starting from antibiotic L 17046 and using n-octyl aldehyde as the reactant carbonyl compound the title compound is obtained as the acetate.

<u>Example 8</u>

Preparation of compounds 8, 9, 10, 11, and 12

By starting from deglucoteicoplanin, i.e. antibiotic L 17392 instead of teicoplanin $A_2$ complex, and following the above procedure of Examples 1, 2, and 3 Compounds 8, 9 and 10 are respectively obtained.

Similarly, but using cyclopentanone and benzaldehyde as carbonyl compounds, Compounds 11 and 12 are respectively prepared. All the title compounds are also isolated as the di-hydrochlorides by substituting glacial acetic acid with hydrochloric acid in the preparation of the eluting mixture acetonitrile:water, at pH 3.0, 1:1 (v/v).

<u>Example 9</u>

By essentially following the procedure described in Example 2 but using teicoplanin $A_2$ factor 2 (2 g) as the teicoplanin starting material, Compound 13 as the acetate is obtained.

<u>Example 10</u>

By essentially following the procedure described in Example 3 but using teicoplanin $A_2$ factor 2 (2 g) as the teicoplanin starting material, Compound 4 as the acetate is obtained.

## Claims

1. A process for preparing $N^{15}$-alkyl derivatives of teicoplanin compounds having the following formula:

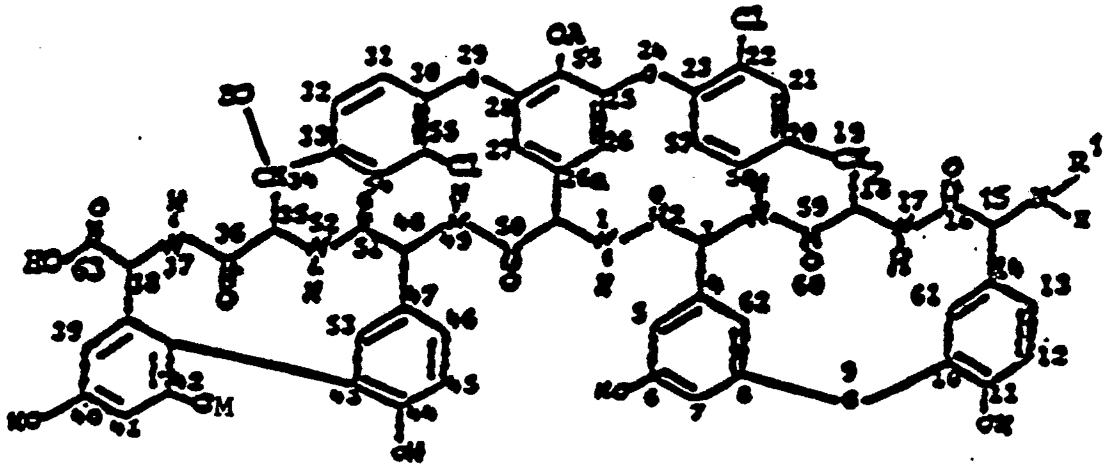

I

wherein

$R^1$ represents hydrogen, $(C_1-C_{12})$alkyl, $(C_4-C_7)$cycloalkyl, cyano $(C_1-C_3)$alkyl, $-(CH_2)_n-OOC-(C_1-C_6)$alkyl, wherein n is an integer selected from 1, 2, 3 and 4; phenyl $(C_1-C_4)$alkyl wherein the phenyl group is optionally substituted in the position ortho, meta and/or para with 1 to 3 groups selected from $(C_1-C_4)$alkyl, nitro, halogen, $(C_1-C_4)$alkoxy, and phenyl;

A represents hydrogen or -N[$(C_9-C_{12})$aliphatic acyl]-beta-D-2-deoxy-2-amino-glucopyranosyl;

B represents hydrogen or N-acetyl-beta-D-2-deoxy-2-amino-glucopyranosyl;

M represents hydrogen or alpha-D-mannopyranosyl; with the proviso that B represents hydrogen only when A and M are simultaneously hydrogen;

and the addition salts thereof, which comprises:

a) adding to a suspension of a teicoplanin compound in an organic polar protic solvent a suitable amount of an alkali metal borohydride at a temperature comprised between 0 °C and 30 °C, then

b) adding a molar excess of a carbonyl compound of at least 20 times with respect to teicoplanin starting material, and

c) reacting the resulting solution with a metal alkali borohydride.

2. A process as in claim 1 further characterized in that the alkali metal borohydride in the step a) is kept under stirring for a time from 30 minutes to 120 minutes before adding the molar excess of the carbonyl compound.

3. A process as in claim 1 or 2 wherein the amount of alkali metal borohydride added renders the pH of the reaction from 8 to 10 as determined by diluting a sample of the non-aqueous reaction mixture with 4 volumes of water.

4. A process as in claim 1 further characterized in that the carbonyl compound is added when the reaction mixture is a clear solution.

5. A process as in claim 1 wherein the carbonyl compound has the same skeleton of substituent $R^1$ and whose oxo group is on the carbon atom which is to link the $N^{15}$amino group of teicoplanin moiety.

6. A process as in claim 1, 2, 3, 4 or 5 wherein the polar protic solvent is an alkanol having from 1 to 4 carbon atoms.

7. A process as in claim 1, 2, 3, 4 or 5 wherein the polar protic solvent is methanol.

8. A process as in claim 1, 2, 3, 4 or 5 wherein the alkali metal borohydride in step a) is sodium borohydride.

9. A process as in claim 1, 2, 3, 4 or 5 wherein the temperature is comprised between 10 and 15 °C.

10. A process as in claim 1, 2, 3, 4, 5, 6, 7, 8, or 9 wherein the excess of the carbonyl compound ranges from 20 times to 100 times with respect to teicoplanin starting material.

11. A process as in claim 1, 2, 3, 4, 5, 6, 7, 8 or 9 wherein the alkali metal borohydride in step c) is selected from sodium borohydride, potassium borohydride and sodium cyanoborohydride.